# EUROPÄISCHE PATENTSCHRIFT

(11) **EP 2 244 994 B1**
(45) Veröffentlichungstag und Bekanntmachung des Hinweises auf die Patenterteilung: **29.10.2014**
(21) Anmeldenummer: 09710882.3
(22) Anmeldetag: 12.02.2009
(51) Int. Cl.: C07C 67/08, C07C 31/125, C07C 29/149, C07C 69/26, C07C 51/09, C07C 53/126

(54) **VERFAHREN ZUM HERSTELLEN VON FETTALKOHOLEN**
METHOD FOR THE PRODUCTION OF FATTY ALCOHOLS
PROCEDE DE PRODUCTION D'ALCOOLS GRAS

(30) Priorität: 13.02.2008 DE 102008008872
(43) Veröffentlichungstag der Anmeldung: 03.11.2010
(73) Patentinhaber: Air Liquide Global E&C Solutions Germany GmbH, 60439 Frankfurt am Main (DE)
(72) Erfinder: BÖNSCH, Rudolf, 55299 Nackenheim (DE); NOWECK, Klaus, 25541 Brunsbüttel (DE)
(86) Internationale Anmeldenummer: PCT/EP2009/000981
(87) Internationale Veröffentlichungsnummer: WO 2009/100902

(56) Entgegenhaltungen:
- WO-A1-94/10112
- US-A- 4 666 879
- D.J. Anneken et al: "Fatty Acids" Ullmann's Encyclopedia of Industrial Chemistry 15. Dezember 2006 (2006-12-15), XP002540225 Gefunden im Internet: URL:http://mrw.interscience.wiley.com/emrw /9783527306732/ueic/article/a10_245/curren t/pdf> [gefunden am 2009-08-03]
- anonymous: "Fatty Acid Technology" 15. Dezember 2006 (2006-12-15), Seiten 1-12, XP002540226 Gefunden im Internet: URL:http://www.lurgi.com/website/fileadmin /user_upload/1_PDF/1_Broshures_Flyer/engli sch/0274e_Fatty_Acid.pdf> [gefunden am 2009-08-03]
- E. SUYENTY ET AL: "Catalyst in Basic Oleochemicals" BULLETIN OF CHEMICAL REACTION ENGINEERING & CATALYSIS, Bd. 2, Nr. 2-3, 19. April 2007 (2007-04-19), Seiten 22-31, XP002540227

## Beschreibung

Die Erfindung betrifft ein Verfahren zum Herstellen von Fettalkoholen, bei dem pflanzliche Öle oder tierische Fette im Gegenstrom zu Dampf bei Temperaturen im Bereich von 220 bis 275° C und bei Drücken im Bereich von 45 bis 65 bar[a] in Fettsäuren und Glycerin gespalten werden, die erzeugte Dispersion physikalisch mittels Schwerkraft oder Fliehkraft in eine Fettsäuren enthaltende Phase und in 12 bis 25 Vol. % Glycerin enthaltendes Absüßwasser getrennt, das Absüßwasser zur Weiterbehandlung ausgeleitet, von der die Fettsäuren enthaltenden Phase zumindest eine Fettsäuren-Fraktion destillativ abgetrennt, die Fettsäuren-Fraktion zusammen mit prozessintern erzeugten Fettalkoholen bei Temperaturen im Bereich von 230 bis 270° C und bei Atmosphärendruck für die Dauer von 6 bis 24 Stunden oder unter Vakuum oder unter Schutzgas bei gleichzeitigem Entfernen des gebildeten Reaktionswassers unter Bildung von Wachsestern in zumindest einer Stufe intensiv vermischt, die gewonnenen eine Säurewertzahl von 1 bis 3 [mg KOH/g] aufweisenden Wachsester an einem aus einer Schüttung von Katalysator-Formkörpern gebildeten Festbett hydriert, indem die Wachsester in dünner Schicht über das Festbett herabrieseln und von der im Gleich- oder Gegenstrom geführten Wasserstoffphase zusammenhängend durchsetzt werden und zu Fettalkoholen reagieren, das Reaktionsprodukt durch Kühlen in Fettalkohole und Wasserstoff getrennt, der Wasserstoff zum Hydrieren der Wachsester in den Prozess rückgeführt, ein Teil der erzeugten Roh-Fettalkohole in einer dem 1,2- bis 1,4-fachen der Menge an Fettsäuren entsprechenden Menge zum Vermischen mit der Fettsäuren-Fraktion in den Prozess rückgeführt und der andere Teil der Roh-Fettalkohole zur Weiterbehandlung ausgeleitet wird.

Aus den Firmenschriften Nr. 274e/3.05/15 und 274e/4.05/10 der Lurgi AG, Frankfurt am Main, ist bekannt, pflanzliche Öle oder tierische Fette in einer Spaltkolonne im

Gegenstrom direkt mit Dampf bei einer Temperatur von 260° C und einem Druck von 55 bar[a] in Kontakt zu bringen und dadurch Fettsäuren und 12 bis 25 Vol. % Glycerin enthaltendes Absüßwasser zu erzeugen. Zum Rückgewinnen des Glycerins werden das von gelösten Fetten und Proteinen gereinigte Absüßwasser durch mehrstufiges Verdampfen des Wassers zu etwa 88 Vol. % Glycerin enthaltendem Roh-Glycerin und ggf. anschließend das Roh-Glycerin destillativ bei einem Vakuum von etwa 15 mbar[a] und einer Temperatur von etwa 160° C zu 92 bis 95 Vol. %-igem Glycerin aufkonzentriert. Die durch Spalten der Öle bzw. Fette erzeugten Fettsäuren werden durch zweistufiges Destillieren in eine Kopffraktion und Mittelfraktion getrennt; wobei sich Monoglyceride, Diglyceride und Salze als Verunreinigungen im Sumpf sammeln. Beide Fettsäure-Fraktionen werden in einem Rührreaktor unter Zusatz von Fettalkohol bei einer Temperatur von etwa 250° C und bei Atmosphärendruck für die Dauer von im Mittel 12 Stunden intensiv vermischt, wobei das Reaktionswasser kontinuierlich abgeführt wird. Dabei werden die Esterbindungen der Triglyceride der Öle bzw. Fette getrennt und es entsteht Wachsester, aus dem anschließend durch Hydrieren bei einem Druck im Bereich von 200 bis 270 bar[a] und einer Temperatur im Bereich von 240 bis 330° C Fettalkohol erzeugt wird. Das beim Verestern anfallende Reaktionsprodukt wird gekühlt und dabei in Wasserstoff und Roh-Fettalkohol getrennt. Der Wasserstoff wird erneut für das Hydrieren des Wachsesters an einem Festbettkatalysator aus Kupfer-Chromoxid-Katalysatorteilchen eingesetzt. Ein Teil des Roh-Fettalkohols wird in den Rührreaktor zur Veresterung der Fettsäuren rückgeführt und der andere Teil, beispielsweise zum Herstellen von Pharma-Glycerin einer Rektifikationskolonne aufgegeben.

Bei dem in der EP-B-0737664 beschriebenen Verfahren zum Herstellen von Fettalkohol wird aus einem Fettsäure enthaltenden flüssigen Ausgangsgemisch bei Temperaturen von 120 bis 320° C und Drücken von 20 bis 400 bar[a] ein zu wenigstens 50 Gew. % aus Wachsester bestehendes flüssiges Zwischenprodukt erzeugt, das durch Zusatz von Wasserstoff und unter Zumischen von feinkörnigem Katalysator hydriert und ein zu mindestens 75 Gew. % aus Fettalkohol bestehendes Rohprodukt gebildet wird. Aus dem Rohprodukt wird ein Fettalkohol enthaltender Teilstrom abgetrennt und dem flüssigen Ausgangsgemisch zugesetzt. Die Hydrierung von Fettsäure-Methylester in der Gasphase erfordert einen separaten Kreislauf.

Gegenstand der DE-C-3425758 ist ein Verfahren zum Herstellen von Alkoholen durch Hydrieren von Verbindungen mit der entsprechenden Anzahl an Kohlenstoffatomen mit einer Säure-, Ester- oder Aldehyd-Funktion, indem die Hydrierung bei einem Wasserstoffdruck im Bereich von 20 bis 100 bar und bei einer Temperatur im Bereich von 150 bis 300° C in Gegenwart eines Katalysators, bestehend aus einer Mischung von Kupfer und Chrom mit etwa 20 bis etwa 40 Gew. % Kupfer (berechnet als Oxid) und einer auf einem Träger befindlichen Kupferkomponente mit etwa 5 bis etwa 45 Gew. % Kupfer, durchgeführt wird.

In der EP-B-0454704 ist ein Verfahren zum Herstellen von Fettalkoholen aus Fettsäure mit einem niederen Alkanol unter Bildung des entsprechenden niederen Esters beschrieben, bei dem der Ester in Gegenwart eines Katalysators bei einer Temperatur im Bereich von 80 bis 140° C und einem Druck im Bereich von 0,1 bis 25 bar zu Fettalkohol hydriert und das Hydrierprodukt unter Einsatz eines sauren Ionenaustauscherkatalysators einer Umesterung unterworfen wird, um den nicht umgesetzten Ester des Hydrierprodukts durch Reaktion mit dem Fettalkohol zu Wachsester umzusetzen. Aus dem Gemisch wird nicht umgesetztes Alkanol verdampft und das verbleibende Gemisch destilliert, so dass als Kopfprodukt ein esterfreier Fettalkohol erzeugt und ein Fettalkohol und Wachsester enthaltender Destillationsrückstand entsteht.

In E. SUYENTY ET AL: "Catalyst in Basic Oleochemicals", BULLETIN OF CHEMICAL REACTION ENGINEERING & CATALYSIS,Bd 2, Nr. 2-3, Seiten 22-31, und WO 94/10112 wird ebenfalls die Hydrierung von aus Fettsäuren und Fettalkoholen gewonnenen Wachsestern an einem CuCr Katalysator offenbart. Der darin offenbarte Katalysator wird jedoch nicht durch Extrusion hergestellt.

In US 4,666,879 wird ein CuCr-Alumina Katalysator zur Hydrierung von Fettsäuremethylestern offenbart. Der Katalysator wird durch Extrusion hergestellt, weist jedoch einen Durchmesser der Poren von über 100 nm von lediglich 0,03 ml/g auf.

Ausgehend von dem in der Beschreibungseinleitung angeführten Stand der Technik liegt der vorliegenden Erfindung die Aufgabe zugrunde, das bekannte Verfahren so zu gestalten, dass der das Hydrieren der Wachsester betreffende Verfahrensabschnitt möglichst einfach ist und dadurch einen kostengünstigeren Verfahrensbetrieb erreicht wird. Insbesondere soll der Verfahrensbetrieb ohne den Einsatz von Hochdruckapparaten und mit vergleichsweise niedrigen Energiekosten möglich werden.

Gelöst ist diese Aufgabe dadurch, dass die Wachsester an einem aus einer Schüttung von gleichförmigen durch Extrusion erzeugten Katalysator-Formkörpern gebildeten Festbett, die als Hauptkomponente Kupfer und Kupferchromoxid und als Nebenkomponenten Zink, Aluminium, Eisen, Silizium und Erdalkali-Elemente enthalten, bei einer Temperatur im Bereich von 180 bis 220° C und bei einem Druck im Bereich von 70 bis 100 bar[a] hydriert werden.

Dabei besitzen die das Festbett bildenden Katalysator-Formkörper relativ große Poren mit einer definierten Porenstruktur aus Makroporen (> 100 nm) von 0,1 bis 0,3 ml/g. Die Kombination aus der sich durch die Porenstruktur ergebenden großen Oberfläche und der Legierungs-Zusammensetzung der Katalysator-Formkörper führt zu einem intensiven Kontakt zwischen den Wachsestern und dem Wasserstoff in der Flüssigkeitsphase, so dass Roh-Fettalkohole mit einer Esterzahl von < 10, insbesondere 3 bis 8, ohne weiteres herstellbar sind. Die bisher für das Hydrieren von Wachsestern eingesetzten das Festbett bildenden Katalysator-Formkörper besitzen eine Porenstruktur, von der nur ein kleiner Teil für die vergleichsweise großen Wachsestermoleküle zugänglich ist und demzufolge das Hydrieren der Wachsester relativ langsam verläuft. Darüber hinaus wird ein geringerer Energieverbrauch erzielt, da keine Kompressorstufen mehr nötig sind. Hinsichtlich der Einsatzstoffe besteht eine relativ große Flexibilität. Das Hydrieren der Wachsester erfolgt erfindungsgemäß bei einem mittleren Druck von 85 bar[a] und einer mittleren Temperatur von 200° C, während beim Einsatz eines herkömmlichen körnigen Kupfer-Chromoxid-Katalysators die entsprechenden Druck- und Temperaturwerte im Mittel bei 250 bar[a] und 270° C liegen. Zusätzlich ist von Vorteil, dass für die Hydrierung der Wachsester im Mittel 50 mol Wasserstoff pro 1 mol Wachsester benötigt werden, währen bei Nutzung eines herkömmlichen körnigen Kupfer-Chromoxid-Katalysators im Mittel 135 mol Wasserstoff pro 1 mol Wachsester in die Hydrierstufe rückgeführt werden müssen. Ferner lässt sich bei Einsatz einer Kaskadenschaltung der für das Hydrieren der Wachsester erforderliche Druck auf einen mittleren Wert von ≤ 50 bar[a] reduzieren und dadurch die zugeführte Menge an Wasserstoff deutlich senken.

In einer besonderen Ausgestaltung der Erfindung besitzen die Katalysator-Formkörper eine Länge von 0,5 bis 6 mm und bestehen aus einem festen Verbund von zwei oder drei oder vier Strängen, wobei der Umkreis des Verbunds einen Durchmesser von 2,5 bis 3,5 mm hat.

Im Rahmen der weiteren Ausgestaltung der Erfindung wird die die Fettsäuren enthaltende Phase einer mehrstufigen, vorzugsweise zweistufigen Destillation unterworfen.

Vorzugsweise wird die die Fettsäuren enthaltende Phase destillativ in eine C₁₂- bis C₁₄-Fettsäuren und eine C₁₆- bis C₁₈-Fettsäuren umfassende Phase getrennt.

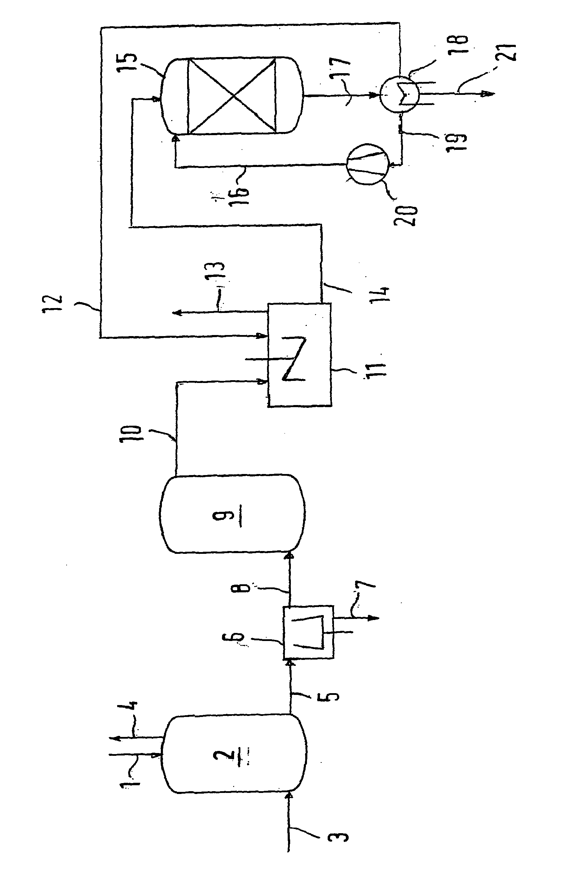

Die Erfindung ist nachstehend durch Ausführungsbeispiele in Verbindung mit einem in der Zeichnung schematisch dargestellten Grundfließbild näher erläutert:
Über Leitung (1) werden der Spaltkolonne (2) am Kopf 1000kg/h Kokosöl, das mit über Leitung (3) zugeführtem einer Temperatur von 250° C aufweisenden Wasserdampf direkt in Kontakt gebracht wird, aufgegeben und in der Spaltkolonne (2) bei einer Temperatur von 235° C und einem Druck von 55 bar[a] in Fettsäuren und Glycerin zerlegt. Am Kopf der Spaltkolonne (2) werden über Leitung (4) leichte flüchtige Verunreinigungen ausgeleitet und einer Weiterbehandlung zugeführt. Über Leitung (5) fließt die erzeugte Dispersion vom Sumpf der Spaltkolonne (2) in eine Zentrifuge (6), in der eine Trennung in Fettsäuren und Glycerin erfolgt. Aus der Zentrifuge (6) werden über Leitung (7) 100 kg Absüßwasser mit einem Gehalt von 18 Vol. % Glycerin zur weiteren Verarbeitung ausgeleitet und über Leitung (8) 900 kg Fettsäuren am Fuß einer Destillationskolonne (9) zugeführt. In der Destillationskolonne (9) wird eine Fraktion von C₁₂- bis C₁₄-Fettsäuren abgetrennt und vom Kopf der Destillationskolonne (9) über Leitung (10) 666 kg Fettsäuren abgezogen und in den Rührreaktor (11) eingeleitet, in dem die Fettsäuren-Fraktion unter Zusatz von über Leitung (12) in einer Menge von 1260 kg eingeleitetem prozessintern erzeugten Fettalkoholen bei einer Temperatur von 255° C für die Dauer von 13 h bei Atmosphärendruck mit Strippen mittels Stickstoff oder unter einem Vakuum von 0,075 bar[a] unter Bildung von Wachsestern intensiv vermischt werden. Das beim Verestern gebildete Reaktionswasser wird über Leitung (13), über die auch das Vakuum erzeugt wird, abgesaugt. Die am Boden das Rührreaktors (11) über Leitung (14) in einer Menge 900 kg abgezogenen einen Säurezahlwert von 2,1 [mg KOH/g] aufweisenden Wachsester werden dem Kopf des Reaktionsbehälters (15), in dem sich ein aus Katalysator-Formkörpern des Typs E 860 (Hersteller: BASF AG, Ludwigshafen) gebildetes Festbett befindet, zusammen mit über Leitung (14) in einer Menge von 50 mol zugeführtem Wasserstoff pro mol Wachsester aufgegeben. Bei einer Temperatur von 195° C und einem Druck von 85 bar[a] werden die Wachsester zu Fettalkoholen umgesetzt. Das über Leitung (17) aus dem Reaktionsbehälter (15) austretende Gemisch wird in dem Kühler (18) in Wasserstoff und Fettalkohole getrennt. Der Wasserstoff wird über Leitung (19) in einen Kompressor (20) geleitet und nach erfolgter Verdichtung in den Reaktionsbehälter (15) rückgeführt, wobei verbrauchter Wasserstoff ergänzt wird. Ein Teil der Fettalkohole wird in einer Menge von 1260 kg über Leitung (12) in den Rührreaktor (11) geleitet und der Rest über Leitung (21) zur Weiterbehandlung ausgeleitet. Üblichweise werden etwa 50 % der Fettalkohole zur Herstellung von Wachsester rückgeleitet und der Rest zur Weiterbehandlung aus dem Prozess ausgeleitet.

## Patentansprüche

1. Verfahren zum Herstellen von Fettalkoholen, bei dem pflanzliche Öle oder tierische Fette im Gegenstrom zu Dampf bei Temperaturen im Bereich von 220 bis 275° C und bei Drücken im Bereich von 45 bis 65 bar[a] in Fettsäuren und Glycerin gespalten werden, die erzeugte Dispersion physikalisch mittels Schwerkraft oder Fliehkraft in eine Fettsäuren enthaltende Phase und in 12 bis 25 Vol. % Glycerin enthaltendes Absüßwasser getrennt, das Absüßwasser zur Weiterbehandlung ausgeleitet, von der die Fettsäuren enthaltenden Phase zumindest eine Fettsäuren-Fraktion destillativ abgetrennt, die Fettsäuren-Fraktion zusammen mit prozessintern erzeugten Fettalkoholen bei Temperaturen im Bereich von 230 bis 270° C und bei Atmosphärendruck für die Dauer von 6 bis 24 h oder unter Vakuum oder unter Schutzgas bei gleichzeitigem Entfernen des gebildeten Reaktionswassers unter Bildung von Wachsestern in zumindest einer Stufe intensiv vermischt, die gewonnenen, eine Säurewertzahl von 1 bis 3 [mg KOH/g] aufweisenden Wachsester an einem aus einer Schüttung von Katalysator-Formkörpern gebildeten Festbett hydriert, indem die Wachester in dünner Schicht über das Festbett herabrieseln und von der im Gleich- oder Gegenstrom geführten Wasserstoffphase zusammenhängend durchsetzt werden und dabei zu Fettalkoholen reagieren, das Reaktionsprodukt durch Kühlen in Fettalkohole und Wasserstoff getrennt, der Wasserstoff zum Hydrieren der Wachsester in den Prozess rückgeführt, ein Teil der erzeugten Roh-Fettalkohole in einer dem 1,2- bis 1,4-fachen der Menge an Fettsäuren entsprechenden Menge zum Vermischen mit der Fettsäuren-Fraktion in den Prozess rückgeführt und der andere Teil der Roh-Fettalkohole zur Weiterbehandlung ausgeleitet wird, **dadurch gekennzeichnet, dass** die Wachsester an einem aus einer Schüttung von gleichförmigen durch Extrusion erzeugten Katalysator-Formkörpern bestehenden Festbett, die als Hauptkomponenten Kupfer und Kupferchromoxid und als Nebenkomponenten Zink, Aluminium, Eisen, Silizium und Erdalkali-Elemente enthalten, und Makroporen (> 100 nm) von 0,1 bis 0,3 ml/g aufweisen, bei einer Temperatur im Bereich von 180 bis 220° C und bei einem Druck im Bereich von 70 bis 100 bar[a] hydriert werden.

2. Verfahren nach Anspruch 1, **dadurch gekennzeichnet, dass** die Katalysator-Formkörper eine Länge von 0,5 bis 6 mm aufweisen und aus einem festen Verbund von zwei oder drei oder vier Strängen bestehen, wobei der Umkreis des Verbunds einen Durchmesser von 2,5 bis 3,5 mm hat.

3. Verfahren nach einem der Ansprüche 1 bis 2, **dadurch gekennzeichnet, dass** die die Fettsäuren enthaltende Phase eine mehrstufigen Destillation unterworfen wird.

4. Verfahren nach Anspruch 3, **dadurch gekennzeichnet, dass** die die Fettsäuren enthaltenden Phase einer zweistufigen Destillation unterworfen wird.

5. Verfahren nach einem der Ansprüche 1 bis 4, **dadurch gekennzeichnet, dass** die die Fettsäuren enthaltende Phase destillativ in eine C₁₂- bis C₁₄-Fettsäuren und in eine C₁₆- bis C₁₈-Fettsäuren umfassende Phase getrennt wird.

## Claims

1. A process for producing fatty alcohols, in which vegetable oils or animal fats are split into fatty acids and glycerol in counterflow to steam at temperatures in the range from 220 to 275°C and at pressures in the range from 45 to 65 bar[a], the dispersion produced is physically separated by means of gravity or centrifugal force into a phase containing fatty acids and into sweet water containing 12 to 25 vol-% glycerol, the sweet water is discharged for further treatment, at least one fatty acid fraction is separated from the phase containing the fatty acids by distillation, the fatty acid fraction together with the fatty alcohols generated in the process is intensively mixed in at least one stage at temperatures in the range from 230 to 270°C and at atmospheric pressure for a period of 6 to 24 hours or under vacuum or under protective gas while simultaneously removing the reaction water obtained by forming wax esters, the wax esters obtained with an acid number of 1 to 3 [mg KOH/g] are hydrogenated on a fixed bed formed of a bed of shaped catalyst bodies, in that the wax esters trickle down over the fixed bed in a thin layer and are continuously permeated by the hydrogen phase guided in cocurrent or countercurrent flow and react to obtain fatty alcohols, the reaction product is separated into fatty alcohols and hydrogen by cooling, the hydrogen for hydrogenating the wax esters is recirculated into the process, part of the raw fatty alcohols produced are recirculated into the process in an amount corresponding to 1.2 to 1.4 times the amount of fatty acids for mixing with the fatty acid fraction and the other part of the raw fatty alcohols is discharged for further treatment, **characterized in that** the wax esters are hydrogenated on a fixed bed consisting of a bed of uniformly shaped catalyst bodies produced by extrusion, which as main components contain copper and copper-chromium oxide and as secondary components zinc, aluminum, iron, silicon and alkaline earth elements, and have macropores (> 100 nm) from 0.1 to 0.3 ml/g, at a temperature in the range from 180 to 220°C and at a pressure in the range from 70 to 100 bar[a].

2. The process according to claim 1, **characterized in that** the shaped catalyst bodies have a length of 0,5 to 6 mm and consist of a firm bond of two or three or four strands, wherein the perimeter of the bond has a diameter of 2.5 to 3.5 mm.

3. The process according to any of claims 1 to 2, **characterized in that** the phase containing the fatty acids is subjected to a multistage distillation.

4. The process according to claim 3, **characterized in that** the phase containing the fatty acids is subjected to a two-stage distillation.

5. The process according to any of claims 1 to 4, **characterized in that** the phase containing the fatty acids is separated by distillation into a phase comprising C₁₂ to C₁₄ fatty acids and a phase comprising C₁₆ to C₁₈ fatty acids.

## Revendications

1. Procédé de production d'alcools gras, dans lequel on sépare des huiles végétales ou des graisses animales à contre-courant de vapeur d'eau à des températures dans la plage de 220 à 275°C et sous des pressions dans la plage de 45 à 64 bar[a] en des acides gras et en de la glycérine, on sépare la dispersion produite, physiquement au moyen de la force de gravité ou de la force centrifuge, en une phase contenant des acides gras et en de l'eau adoucie contenant de 12 à 25 % en volume de glycérine, on envoie l'eau adoucie à un traitement ultérieur, on sépare par distillation au moins une fraction d'acides gras de la phase contenant des acides gras, on mélange intensément en au moins un stade la fraction d'acides gras ensemble avec des alcools gras produits d'une manière interne à l'opération à des températures de l'ordre de 230 à 270°C et sous la pression atmosphérique pendant une durée de 6 à 24 h ou sous vide ou sous protection gazeuse en éliminant simultanément l'eau de réaction formée avec formation d'esters de cire, on hydrure les esters de cire obtenus ayant un indice d'acide de 1 à 3 [mg KOH/g] sur un lit fixe formé d'un amas de corps façonnés de catalyseur en faisant ruisseler les esters de cire en couche mince sur le lit fixe et en les faisant traverser sans interruption par la phase d'hydrogène en courant de même sens ou à contre-courant et en les faisant réagir ainsi pour donner des alcools gras, on sépare le produit de réaction par refroidissement en des alcools gras et de l'hydrogène, on retourne l'hydrogène à l'opération pour l'hydruration des esters de cire, on retourne à l'opération une partie des alcools gras bruts en une quantité correspondant de 1,2 à 1,4 fois à la quantité d'acides gras pour le mélange à la fraction d'acides gras et on envoie l'autre partie des alcools gras bruts pour le traitement ultérieur, **caractérisé en ce qu'**on hydrure à une température dans la plage de 180 à 220°C sous une pression dans la plage de 70 à 100 bar[a], les esters de cire sur un lit fixe constitué d'un amas de corps façonnés de catalyseur de même forme produits par extrusion, qui contiennent comme constituants principaux du cuivre et de l'oxyde de cuivre et de chrome, et comme constituants secondaires du zinc, de l'aluminium, du fer, du silicium et des éléments de métaux alcalinoterreux et qui ont des macropores (> 100 nm) de 0,1 à 0,3 ml/g.

2. Procédé suivant la revendication 1, **caractérisé en ce que** les corps façonnés de catalyseur ont une longueur de 0,5 à 6 mm et sont constitués en un composite solide de deux ou de trois ou de quatre brins, le pourtour du composite ayant un diamètre de 2,5 à 3,5 mm.

3. Procédé suivant l'une des revendications 1 et 2, **caractérisé en ce qu'**on soumet à une distillation à plusieurs étages la phase contenant les acides gras.

4. Procédé suivant la revendication 3, **caractérisé en ce qu'**on soumet à une distillation à deux étages la phase contenant les acides gras.

5. Procédé suivant l'une des revendications 1 à 4, **caractérisé en ce qu'**on sépare la phase contenant des acides gras par distillation en une phase comprenant des acides gras en C₁₂ à C₁₄ et une phase comprenant des acides gras en C₁₆ à C₁₈.
